(19) Europäisches Patentamt / European Patent Office / Office européen des brevets

(11) **EP 2 921 163 B1**

(12) **EUROPEAN PATENT SPECIFICATION**

(45) Date of publication and mention
of the grant of the patent:
**10.10.2018 Bulletin 2018/41**

(21) Application number: **13855653.5**

(22) Date of filing: **07.11.2013**

(51) Int Cl.:
*A61K 8/39* *(2006.01)*      *A61K 8/06* *(2006.01)*
*A61K 8/37* *(2006.01)*      *A61K 8/41* *(2006.01)*
*A61K 8/49* *(2006.01)*      *A61Q 17/04* *(2006.01)*

(86) International application number:
**PCT/JP2013/080065**

(87) International publication number:
**WO 2014/077173 (22.05.2014 Gazette 2014/21)**

(54) **OIL-IN-WATER EMULSION COMPOSITION**

ÖL-IN-WASSER-EMULSIONSVERBINDUNG

COMPOSITION DE TYPE ÉMULSION HUILE DANS EAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.11.2012 JP 2012250129**

(43) Date of publication of application:
**23.09.2015 Bulletin 2015/39**

(73) Proprietor: **Kao Corporation
Chuo-Ku
Tokyo 103-8210 (JP)**

(72) Inventor: **MURATA, Takeshi
Odawara-shi
Kanagawa 250-0002 (JP)**

(74) Representative: **Hoffmann Eitle
Patent- und Rechtsanwälte PartmbB
Arabellastraße 30
81925 München (DE)**

(56) References cited:
**EP-A1- 2 543 356      WO-A1-2011/155404
WO-A1-2013/161429      WO-A2-2007/122822
JP-A- H01 266 844      JP-A- H07 291 827
JP-A- H10 194 923      JP-A- 2007 031 336
JP-A- 2011 195 478      JP-A- 2012 001 527**

Remarks:
The file contains technical information submitted after
the application was filed and not included in this
specification

## Description

Field of the Invention

The present invention relates to an oil-in-water emulsion composition stably containing, in an oil phase, an ultraviolet absorber which is solid at room temperature.

Background of the Invention

Heretofore, preparations which contain an ultraviolet absorber such as a p-methoxycinnamic acid ester or oxybenzone and have an ultraviolet protective effect have been used as sunscreen skin preparations for external use (see Patent Literature 1).

It has been pointed out in recent years that ultraviolet rays in the UV-A region (320 to 400 nm) reach the deep part of the skin and become principally responsible for inducing photoaging or skin cancer. As for cosmetics, there has also been a growing demand for a protective effect against ultraviolet rays in the UV-A region.

However, currently utilized ultraviolet absorbers having a high protective effect against ultraviolet rays in the UV-A region, such as diethylamino hydroxybenzoyl hexyl benzoate and t-butyl methoxydibenzoylmethane, are solid at 25°C. These ultraviolet absorbers are poorly soluble in water or nonpolar oils and thus tend to present problems such as their deposition. In addition, the ultraviolet absorbers present problems associated with a feeling upon use, such as stickiness and poor spreadability (see Patent Literature 2).

The dissolution of these ultraviolet absorbers requires mixing them with large amounts of highly polar oils. This may cause problems associated with usability, such as a strong feeling of stickiness and unevenness during application, and further, problems associated with stability, such as the deposition of the ultraviolet absorbers at low temperatures.

In order to solve such problems, for example, the optimization of the dispersibility of ultraviolet absorbers or highly polar oils has been practiced by use of several types of surfactants in combination (see Patent Literature 3).

Citation List

Patent Literature

Patent Literature 1: JP-B-3491933
Patent Literature 2: JP-A-2008-162988
Patent Literature 3: WO 2007/122822
Patent Literature 4: WO 2011/136121
Patent Literature 5: WO 2011/155404

WO 2013/161429 is a post-published document relating to a liquid aryl group-containing polyorganosiloxane comprising an arylsiloxy unit of a specific formula, and the use thereof in cosmetic compositions.

EP 2 543 356 is a post-published document concerning a to a sunscreen composition comprising (a) a surfactant, (b) water, (c) an oil component, and (d) a water soluble ultraviolet absorbent and/or oil soluble ultraviolet absorbent that is in the bicontinuous microemulsion phase at 25°C.

JP 2007-031336 (A) is directed to a water-in-oil type skin external preparation comprising a a polyglycerin derivative of a specific formula, which is said to be is excellent in emulsification stability, and particularly is improved in the emulsification stability when a polar oil or a silicone oil is incorporated.

JP 2012-001527 concerns a skin preparation for external use, which contains the following components (A), (B) and (C): (A) a polyoxyethylene alkyl or alkenyl ether that has an alkyl group or alkenyl group with 20-24 carbon atoms, while having an average mole number of added ethylene oxide of 1.5-4; (B) a water-soluble polymer; and (C) a skin activating ingredient.

JP H01 266844 seeks to improve the emulsified state of an emulsified composition and the stability over a long period by blending an adduct of polyoxyethylene to a higher alcohol having 22 or more carbon atoms with lower alcohol and an oil component to prepare the emulsified composition.

JP 2011-195478 relates to an oil-in-water sunscreen cosmetic, which contains (a) a water-soluble polymer, (b) a nonionic surfactant having HLB of 8 or less, (c) an anionic surfactant, (d) a lipo-soluble ultraviolet light absorber and (e) polyhydric alcohol.

JP H10-194923 concerns an emulsified composition obtained by adding a metallic soap to propolis extract or adding a metallic soap and glycerol fatty acid ester to propolis extract. The emulsified composition is useful as a cosmetic such as cream, milk lotion, pack or rinse applied to skin, scalp, hair, etc.

JP H07-291827 describes the preparation of an O/W-type skin cream from an oily phase component and an aqueous phase component, wherein the oily phase component is subjected to mixing dissolution treatment at low temperature under reduced pressure, the treated oily phase component is mixed with the aqueous phase component and the mixture is emulsified at a low temperature.

Summary of the Invention

The present invention provides an oil-in-water emulsion composition comprising the following components (A) to (C):

   (A) a polyoxyethylene alkyl or alkenyl ether which has an alkyl or alkenyl group having 20 or more and 24 or less carbon atoms and has an average molar number of ethylene oxide added of 1.5 or more and 4 or less,
   (B) a polar oil which has an IOB value of 0.2 or more and 0.45 or less and is liquid at 25°C, provided that a silicone oil having a silicon backbone is excluded, and
   (C) an ultraviolet absorber which is solid at 25°C,

wherein the content mass ratio of the component (A) to the component (B), (A/B), is 0.01 or more and 1.8 or less.

The present invention also provides the oil-in-water emulsion composition for external use as a sun screen preparation and a method for producing the oil-in-water emulsion. The invention is defined by the appended claims.

Brief Description of the Drawing

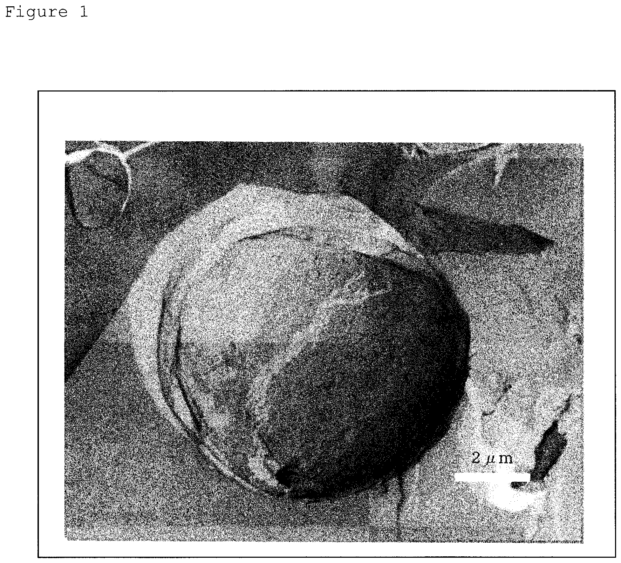

[Figure 1] Figure 1 is an electron microscope photograph of a structure present on the emulsified particle interface (oil phase interface) of an oil-in-water emulsion composition of Example 2.

Embodiments for Carrying out the Invention

The oil-in-water emulsion composition described in Patent Literature 3 is improved to some extent in terms of stability. This composition, however, is limited by components that can be contained therein, and has small variations of formulations. When containing a large amount of a solid ultraviolet absorber, the composition has unsatisfactory stability at high temperatures and low temperatures and may cause deposition of the ultraviolet absorber or the like, resulting in the problem of stability over time.

Thus, the present invention provides an oil-in-water emulsified cosmetic composition which is excellent in the stability of a solid oil-soluble ultraviolet absorber contained, is agreeable to the skin, offers a reduced feeling of friction, exhibits excellent stability over time, and has a high moisturizing effect.

The present inventor found that a significant water evaporation inhibiting effect is obtained by use of a polyoxyethylene alkyl or alkenyl ether which has an alkyl or alkenyl group having 20 to 24 carbon atoms and has an average molar number of ethylene oxide added of 1.5 to 4 (hereinafter, this ether is referred to as polyoxyethylene alkyl ether (A)) for an aqueous composition in combination with a water-soluble polymer (Patent Literature 3). When the polyoxyethylene alkyl ether (A) is mixed into an oil phase containing a specific polar oil, the water evaporation inhibiting effect is disadvantageously reduced (Patent Literature 4). Accordingly, the present inventor conducted detailed study on the behavior of an oil agent and the polyoxyethylene alkyl ether (A) in the oil phase and consequently found that the polyoxyethylene alkyl ether (A) is present on the interface between the aqueous phase and the oil phase so as to form a soft structure. The present inventor further found that when a poorly soluble ultraviolet absorber is contained in this oil phase, an oil-in-water emulsion composition which prevents the ultraviolet absorber from being deposited, is excellent in emulsion stability, is highly agreeable to the skin, offers reduced feelings of stickiness and friction, and exhibits an excellent moisturizing effect after application is obtained by virtue of the presence of this structure. On the basis of these findings, the present invention was completed.

The oil-in-water emulsion composition of the present invention is a homogeneous emulsion composition stably containing, in an oil phase, an ultraviolet absorber which is solid at 25°C. The oil-in-water emulsion composition of the present invention is agreeable to the skin, offers a reduced feeling of friction, and is excellent in stability over time and moisturizing effect. The oil-in-water emulsion composition of the present invention can be preferably used for sunburn prevention.

Hereinafter, the constitution of the present invention will be described in detail.

The IOB value according to the present invention is an abbreviation of inorganic/organic balance. This value corresponds to the ratio of the inorganic value of a compound to the organic value of the compound and serves as an index for showing the degree of polarity of an organic compound. Specifically, the value is represented by IOB value = Inorganic value / Organic value.

In this context, the "inorganic value" and the "organic value" are respectively set according to various atoms or functional groups such that, for example, the "organic value" is 20 for one carbon atom in the molecule and the "inorganic value" is 100 for one hydroxy group in the molecule. The IOB value of an organic compound is calculated by the summation of the "inorganic values" and the "organic values" of all atoms and functional groups in the organic compound (see e.g., Yoshio Koda, "Yuki Gainenzu - Kiso to Oyo - (Organic Conceptual Diagram - Basis and Application - in English)", p. 11-17, Sankyo Publishing Co., Ltd., published in 1984).

The term "liquid" described in the present invention refers to a state having fluidity at 25°C and includes paste forms. Also, the term "solid" refers to a state having no fluidity at 25°C.

The oil-in-water emulsion composition of the present invention (Example 2) was observed using a transmission electron microscope under conditions given below. As a result, a layer structure as shown in Figure 1 was confirmed on the surface of emulsified particles (oil phase interface) having a diameter of approximately 6 $\mu$m. Thus, the structure according to the present invention refers to such a layer structure locally present on the oil phase interface. Also, this layer structure was found by X-ray analysis to be a multilamellar vesicle formed by the polyoxyethylene alkyl ether (A).

[Measurement conditions]

Apparatus: transmission electron microscope (model JEM-1011 manufactured by JEOL Ltd.)
Condition: acceleration voltage of 100 kV
Sample preparation: freeze-fracture replica technique
Sample preparation apparatus: model JFD-9010 manufactured by JEOL Ltd.

The polyoxyethylene alkyl or alkenyl ether (A) used in the present invention has an alkyl or alkenyl group having 20 or more and 24 or less carbon atoms and an average molar number of ethylene oxide added of 1.5 or more and 4 or less.

The alkyl or alkenyl group in the polyoxyethylene alkyl ether (A) may be linear or branched and is not limited by its structure. The alkyl or alkenyl group is preferably a linear or branched alkyl group, more preferably a linear alkyl group. The number of carbon atoms of the alkyl or alkenyl group is 20 or more and 24 or less, preferably 21 or more and 23 or less. A behenyl group having 22 carbon atoms is more preferred. When the number of carbon atoms of the alkyl or alkenyl group is less than 20, the resulting polyoxyethylene alkyl ether (A) fails to be present on the oil phase interface so as to form a structure. The number of carbon atoms of the alkyl or alkenyl group exceeding 24 is not preferred for formulation because the resulting polyoxyethylene alkyl ether (A) is difficult to dissolve in the oil phase.

The average molar number of ethylene oxide added in the polyoxyethylene alkyl ether (A) is in the range of 1.5 or more and 4 or less, preferably 1.5 or more and 3 or less, more preferably 1.5 or more and 2.5 or less. An average molar number of ethylene oxide added less than 1.5 is not preferred because the resulting polyoxyethylene alkyl ether (A) is highly crystalline and is thus difficult to dissolve in the oil phase. Alternatively, when the average molar number of ethylene oxide added exceeds 4, the resulting polyoxyethylene alkyl ether (A) is less likely to form a structure on the oil phase interface. Generally available polyoxyethylene alkyl ether (A) is a mixture having a very wide distribution centered on the desired degree of polymerization as to the molar number of ethylene oxide added. For the present invention, it is important to use the polyoxyethylene alkyl ether (A) in which the average molar number of ethylene oxide added falls within the range mentioned above.

Examples of the polyoxyethylene alkyl ether (A) of the present invention include polyoxyethylene (2) arachyl ether, polyoxyethylene (3) arachyl ether, polyoxyethylene (4) arachyl ether, polyoxyethylene (2) behenyl ether, polyoxyethylene (3) behenyl ether, polyoxyethylene (4) behenyl ether, polyoxyethylene (2) carnaubyl ether, polyoxyethylene (3) carnaubyl ether, and polyoxyethylene (4) carnaubyl ether, and preferably include polyoxyethylene (2) behenyl ether, polyoxyethylene (3) behenyl ether, and polyoxyethylene (4) behenyl ether. In this context, the polyoxyethylene alkyl ether (A) of the present invention may be used in combination with a polyoxyethylene alkyl ether other than those exemplified above as long as the average molar number of ethylene oxide added in the polyoxyethylene alkyl ether (A) used falls within the range.

The content of the polyoxyethylene alkyl ether (A) in the oil-in-water emulsion composition of the present invention with respect to the total amount of the composition is preferably 0.01% by mass or more, more preferably 0.1% by mass or more, even more preferably 0.5% by mass or more, and is preferably 15% by mass or less, more preferably 10% by mass or less, even more preferably 5% by mass or less. The specific range is preferably from 0.01 to 15% by mass, more preferably from 0.1 to 10% by mass, even more preferably from 0.5 to 5% by mass. This range is preferred because the resulting polyoxyethylene alkyl ether (A) tends to form a structure on the oil phase interface and produces an excellent moisturizing effect.

The polar oil (B) used in the present invention is a liquid oil agent having an IOB value in the range of 0.2 or more and 0.45 or less, provided that the polar oil (B) excludes a silicone oil having a silicon backbone. Particularly, the polar oil (B) is preferably a liquid polar oil having an IOB value of 0.22 or more and 0.45 or less, because the polyoxyethylene alkyl ether (A) tends to form a structure on the oil phase interface and the ultraviolet absorber which is solid at 25°C is highly soluble.

Specific examples of the component (B) can include one or two or more selected from the group consisting of propylene glycol isostearate (IOB value: 0.4), diethylhexyl sebacate (IOB value: 0.24), 2-ethylhexyl p-methoxycinnamate (IOB value: 0.28), trimethylpropane tri-2-ethylhexanoate (IOB value: 0.31), di-2-ethylhexyl succinate (IOB value: 0.32), propylene glycol di(caprylate-caprate) (IOB value: 0.32), 2-ethylhexyl 2-cyano-3,3-diphenylacrylate (IOB value: 0.33), glyceryl tri(caprylate-caprate) (IOB value: 0.33), trimethylolpropane trioctanoate (IOB value: 0.33), glyceryl tricaprylate (IOB value: 0.33), ethylene glycol dioctanoate (IOB value: 0.35), glyceryl dimyristate (IOB value: 0.35), diethylene glycol dilaurate (IOB value: 0.35), pentaerythritol tetra-2-ethylhexanoate (IOB value: 0.35), glyceryl tri-2-ethylhexanoate (IOB value: 0.35), glyceryl monostearate diacetate (IOB value: 0.36), octyldodecyl lactate (IOB value: 0.36), propylene glycol monostearate (IOB value: 0.38), propylene glycol oleate (IOB value: 0.39), oleyl lactate (IOB value: 0.39), propylene glycol dicaproate (IOB value: 0.4), diisopropyl sebacate (IOB value: 0.4), ethylene glycol monostearate (IOB value: 0.4), diethylene glycol dicaprate (IOB value: 0.41), glyceryl di(coconut oil fatty acid) (IOB value: 0.41), glyceryl dilaurate (IOB value: 0.41), glyceryl sesquioleate (IOB value: 0.41), ethylene glycol monooleate (IOB value: 0.41), coconut oil alcohol (IOB value: 0.42), lauryl alcohol (IOB value: 0.42), cetyl lactate (IOB value: 0.42), diethyl sebacate (IOB value: 0.43), castor oil fatty acid methyl ester (IOB value: 0.43), ethylene glycol palmitate (IOB value: 0.44), and polyethylene glycol dilaurate (IOB value: 0.45).

Of them, one or two or more selected from the group consisting of 2-ethylhexyl p-methoxycinnamate, propylene glycol isostearate, diethylhexyl sebacate, diisopropyl sebacate, and glyceryl tri-2-ethylhexanoate are preferably used in terms of the easy formation of the structure on the oil phase interface. 2-Ethylhexyl p-methoxycinnamate and/or diisopropyl sebacate is more preferred because the ultraviolet absorber which is solid at 25°C is prevented from being deposited.

The content of the component (B) with respect to the total amount of the composition is preferably 0.125% by mass or more, more preferably 0.25% by mass or more, even more preferably 1.25% by mass or more, and is preferably 30% by mass or less, more preferably 25% by mass or less, and even more preferably 20% by mass or less, in terms of the solubility of the ultraviolet absorber which is solid at 25°C and in terms of reduction in feelings of stickiness and friction. The specific range is preferably from 0.125 to 30% by mass, more preferably from 0.25 to 25% by mass, even more preferably from 1.25 to 20% by mass. The component (B) having the content in this range can dissolve a sufficient amount of the ultraviolet absorber which is solid at 25°C, produces excellent UVA protective ability, and offers a favorable feeling upon use with reduced feelings of stickiness and friction.

In the present invention, a liquid oil other than the component (B) may be contained in the composition. In this case, the content of the component (B) with respect to the total amount of the liquid oils is preferably 10% by mass or more, more preferably 30% by mass or more, even more preferably 50% by mass or more, and is, specifically, preferably from 10 to 100% by mass, more preferably from 30 to 100% by mass, even more preferably from 50 to 100% by mass, because the polyoxyethylene alkyl ether (A) tends to form a structure on the oil phase interface.

The content mass ratio of the component (A) to the component (B), (A/B), is 0.01 or more, preferably 0.03 or more, even more preferably 0.04 or more, and is 1.8 or less, preferably 0.7 or less, more preferably 0.5 or less, even more preferably 0.4 or less, in terms of an occluding effect, a feeling of stickiness, a feeling of friction, and stability over time. The specific range is from 0.01 to 1.8, preferably from 0.01 to 0.7, more preferably from 0.03 to 0.7, more preferably from 0.03 to 0.5, even more preferably from 0.04 to 0.4.

The ultraviolet absorber which is solid at 25°C (C) used in the present invention is not particularly limited as long as the ultraviolet absorber can be contained in cosmetics, quasi-drugs, or pharmaceutical drugs.

Specific examples of the component (C) can include one or two or more selected from the group consisting of 2-ethylhexyl dimethoxybenzylidene dioxoimidazolidine propionate, diethylamino hydroxybenzoyl hexyl benzoate, 2,4-bis{[4-(2-ethylhexyloxy)-2-hydroxy]-phenyl}-6-(4-methoxyphenyl)-1,3,5-triazine, 2,4,6-tris[4-(2-ethylhexyloxycarbonyl)anilino]-1,3,5-triazine, and 4-tert-butyl-4'-methoxydibenzoylmethane.

Of them, one or two or more selected from the group consisting of diethylamino hydroxybenzoyl hexyl benzoate, 2,4-bis{[4-(2-ethylhexyloxy)-2-hydroxy]-phenyl}-6-(4-methoxyphenyl)-1,3,5-triazine, and 2,4,6-tris[4-(2-ethylhexyloxycarbonyl)anilino]-1,3,5-triazine can be preferably used in terms of water resistance and a feeling upon use.

In general, the component (C) is commercially available. Examples thereof can include Soft Shade DH (2-ethylhexyl dimethoxybenzylidene dioxoimidazolidine propionate; manufactured by Ajinomoto Co., Inc.), Uvinul A Plus (2-(4-diethylamino-2-hydroxybenzoyl) hexyl benzoate; manufactured by BASF Japan Ltd.), Tinosorb S (2,4-bis{[4-(2-ethylhexyloxy)-2-hydroxy]-phenyl}-6-(4-methoxyphenyl)-1,3,5-triazine; manufactured by BASF Japan Ltd.), Uvinul T-150 (2,4,6-tris[4-(2-ethylhexyloxycarbonyl)anilino]-1,3,5-triazine), and PARSOL 1789 (4-tert-butyl-4'-methoxydibenzoylmethane; manufactured by F. Hoffmann-La Roche, Ltd.).

The content of the component (C) used in the present invention with respect to the total amount of the composition is preferably 0.5% by mass or more, and is preferably 10% by mass or less, more preferably 7.5% by mass or less, even more preferably 5% by mass or less. The specific range is preferably from 0.5 to 10% by mass, more preferably from 0.5 to 7.5% by mass, even more preferably from 0.5 to 5% by mass. The component (C) having the content in this range produces excellent ultraviolet protective ability and offers a favorable feeling upon use as to spreadability and staying property.

The content mass ratio of the component (B) to the component (C), (B/C), is preferably 2 or more, more preferably 2.2 or more, even more preferably 2.4 or more, and is preferably 20 or less, more preferably 10 or less, more preferably 7 or less, and even more preferably 4 or less, from the viewpoint of preventing the deposition of the ultraviolet absorber, reducing a feeling of stickiness and a feeling of friction, and producing a high UV protective effect. The specific range is preferably from 2 to 20, more preferably from 2.2 to 10, more preferably from 2.2 to 7, more preferably from 2.4 to 7, even more preferably from 2.4 to 4.

In the present invention, the polyoxyethylene alkyl ether (A) forms a structure on the oil phase interface, while the component (C) is dissolved to reside in a solution of the component (B). Thus, the component (C) originally having the property of being easily deposited is probably maintained stably in the oil phase. From such a viewpoint, the component (B) in a small amount relative to that of the component (C) prevents the deposition of the component (C) and offers a favorable feeling upon use.

In the present invention, preferably, a water-soluble polymer (D) is further used for enhancing the dispersion stability of the oil phase. Examples of the water-soluble polymer used in the present invention include water-soluble cationic polymers, anionic polymers, nonionic polymers, and amphoteric polymers or bipolar polymers.

Specific examples of the cationic polymers include hydroxyethylcellulose having a O-[2-hydroxy-3-(trimethylammonio)propyl] chloride group (polyquaternium-10), (vinylpyrrolidone-dimethylaminomethylethyl methacrylate copolymer diethyl sulfate (polyquaternium-11), and methylvinylimidazolium chloride-vinylpyrrolidone copolymer.

Specific examples of the anionic polymers include carboxyvinyl polymer, carboxymethylcellulose, carrageenan, xanthan gum, polystyrene sulfonate, agar, ghatti gum, karaya gum, pectin, alginate salt, acrylic acid-alkyl methacrylate copolymer, (sodium acrylate/sodium acryloyldimethyl taurate) copolymer, acrylic acid or methacrylic acid derivatives such as alkali metal and ammonium salts of poly(acrylic acid) or acrylic acid or methacrylic acid, and hyaluronic acid or alkali metal salts thereof.

Specific examples of the nonionic polymers include cellulose ether such as hydroxybutylmethylcellulose, hydroxypropylcellulose, hydroxypropylmethylcellulose, ethylhydroxyethylcellulose, and hydroxyethylcellulose, propylene glycol alginate, polyacrylamide, poly(ethylene oxide), polyvinyl alcohol, polyvinyl pyrrolidone, hydroxypropyl guar gum, locust bean gum, amylose, hydroxyethyl amylose, starch and starch derivatives, and mixtures thereof.

Specific examples of the amphoteric polymers or bipolar polymers include octylacrylamide/acrylate/butylaminoethyl methacrylate copolymer, polyquaternium-47, and polyquaternium-43.

These water-soluble polymers can be used singly or in appropriate combination of two or more thereof. In terms of easy applicability to various formulations, preferred examples thereof include carboxyvinyl polymer, acrylic acid-alkyl methacrylate copolymer, xanthan gum, hydroxypropylmethylcellulose, polyacrylamide, (sodium acrylate/sodium acryloyldimethyl taurate) copolymer, hyaluronic acid or an alkali metal salt thereof.

The content of the component (D) with respect to the total amount of the composition is preferably 0.01% by mass or more, more preferably 0.05% by mass or more, and is preferably 5% by mass or less, more preferably 3% by mass or less. The specific range is preferably from 0.01 to 5% by mass, more preferably from 0.05 to 3% by mass. The content in the range is preferred because the stability of the preparation is maintained without a feeling of stickiness attributed to the water-soluble polymer.

In the present invention, preferably, the oil-in-water emulsion composition further comprises (E) a saturated monohydric alcohol having 1 to 3 carbon atoms, from the viewpoints of enhancing spreadability during application, reducing a feeling of stickiness, and enhancing water resistance and perspiration resistance. Specific examples of the component (E) include methyl alcohol, ethyl alcohol, propyl alcohol, and isopropyl alcohol.

The content of the component (E) is not particularly limited. The content of the component (E) with respect to the total amount of the composition is preferably 1% by mass or more, more preferably 3% by mass or more, even more preferably 5% by mass or more, and is preferably 25% by mass or less, more preferably 20% by mass or less, and even more preferably 15% by mass or less, from the viewpoint of enhancing spreadability during application, reducing a feeling of stickiness, and improving water resistance and perspiration resistance. The specific range is preferably from 1 to 25% by mass, more preferably from 3 to 20% by mass, even more preferably from 5 to 15% by mass.

In the present invention, preferably, the oil-in-water emulsion composition further comprises (F) a polyhydric alcohol in order to enhance moisturizing properties and spreadability during application. Examples of the component (F) include: glycols such as ethylene glycol, diethylene glycol, triethylene glycol, polyethylene glycol (average molecular weight: lower than 650), propylene glycol, dipropylene glycol, polypropylene glycol (average molecular weight: lower than 650), isoprene glycol, and 1,3-butylene glycol; glycerin, diglycerin, and polyglycerin. Of them, ethylene glycol, diethylene glycol, propylene glycol, dipropylene glycol, or 1,3-butylene glycol can be preferably used. Dipropylene glycol is more preferred. These polyhydric alcohols can be used singly or in appropriate combination of two or more thereof.

The content of the component (F) with respect to the total amount of the composition is preferably 0.1% by mass or more, more preferably 0.5% by mass or more, even more preferably 1% by mass or more, and is preferably 30% by mass or less, more preferably 20% by mass or less, more preferably 15% by mass or less, even more preferably 10% by mass or less, from the viewpoint of enhancing moisturizing properties and spreadability during application. The specific range is preferably from 0.1 to 30% by mass, more preferably from 0.5 to 20% by mass, more preferably from 0.5 to 15% by mass, more preferably from 1 to 15% by mass, even more preferably from 1 to 10% by mass.

In the oil-in-water emulsion composition of the present invention, the content of water with respect to the total amount of the composition is preferably 40% by mass or more, more preferably 45% by mass or more, and is preferably 80% by mass or less, more preferably 75% by mass or less, from the viewpoint of forming an oil-in-water emulsion composition excellent in stability over time. The specific range is preferably from 40 to 80% by mass, more preferably from 45 to 75% by mass.

In the oil-in-water emulsion composition of the present invention, various components usually contained in cosmetics, for example, surfactants, oil components, silicone compounds, higher alcohols, fluorine compounds, resins, thickeners, antibacterial or antiseptic agents, fragrances, moisturizers, salts, solvents, antioxidants, chelating agents, neutralizing agents, pH adjusters, insect repellents, and biologically active components, can be used in addition to the components mentioned above and water, within the range in which the effects of the present invention are not impaired.

The oil-in-water emulsion composition of the present invention is not particularly limited by its uses and can be preferably used in cosmetic compositions, pharmaceutical drugs, quasi-drugs, and the like. The emulsion composition of the present invention is preferably used as a skin preparation for external use of type which is retained on the skin without being washed off, because of its excellent moisture-occluding effect. The emulsion composition of the present invention is preferably used for sunburn prevention and is more preferably applied to sunscreens, suntan preparations, makeup base cosmetic compositions, foundations having ultraviolet protective ability, and the like.

The formulation of the oil-in-water emulsion composition of the present invention is applicable to, for example, liquid, emulsion, cream, paste, solid, and multilayer forms and is also applicable to sheet preparations, spray preparations, and mousse preparations.

The oil-in-water emulsion composition of the present invention can prevent the component (C) ultraviolet absorber which is solid at 25°C from being deposited, enhance the dispersibility of the oil phase, and produce excellent stability over time by containing the components (A) to (C) in the oil phase. As for the structure of the emulsified particles in the oil-in-water emulsion composition of the present invention, it appears that the component (A) is present on the emulsified particle (oil phase) interface, as described above, so as to form a soft structure (multilamellar vesicle structure), and the component (C) is stably dissolved in the component (B) in the oil phase. Such a structure formed prevents the ultraviolet absorber from being deposited, is excellent in emulsion stability, is highly agreeable to the skin, offers reduced feelings of stickiness and friction, and exhibits a favorable moisturizing effect after application.

In order to enhance the orientation of the polyoxyethylene alkyl ether (A) toward the oil phase interface, the oil-in-water emulsion composition of the present invention is preferably produced by dissolving an oil-phase component containing the components (A) to (C) by heating at a temperature exceeding 45°C and emulsifying the oil-phase component with an aqueous-phase component of 15 to 40°C. More specifically, the components (A) to (C) are dissolved, together with other oil-phase components, by heating at a temperature exceeding 45°C, preferably a temperature of 50 to 80°C, and the mixture is uniformly stirred and mixed. Then, the oil-phase component is uniformly mixed with an aqueous-phase component of 15 to 40°C, preferably 20 to 30°C, by a method such as phase-inversion emulsification or non-phase-inversion emulsification to form an oil-in-water emulsion composition. This emulsification is preferably performed by the gradual addition of the oil-phase component into the aqueous-phase component. More preferably, the uniformly stirred oil phase is gradually added into the aqueous phase (including the component (D)), and the mixture is uniformly stirred and mixed, and then cooled to room temperature to produce an emulsion composition. The polyoxyethylene alkyl ether (A) forms a structure on the oil phase interface at the same time with the addition of the oil phase into the aqueous phase. As a result, a stable oil-in-water emulsion composition can be obtained.

In relation to the embodiments mentioned above, the present invention further discloses the following embodiments:

<1> An oil-in-water emulsion composition comprising the following components (A) to (C):

(A) a polyoxyethylene alkyl or alkenyl ether which has an alkyl or alkenyl group having 20 or more and 24 or less carbon atoms and has an average molar number of ethylene oxide added of 1.5 or more and 4 or less,
(B) a polar oil which has an IOB value of 0.2 or more and 0.45 or less and is liquid at 25°C, provided that a silicone oil having a silicon backbone is excluded, and
(C) an ultraviolet absorber which is solid at 25°C,

wherein the content mass ratio of the component (A) to the component (B), (A/B), is 0.01 or more and 1.8 or less.

<2> The oil-in-water emulsion composition according to <1>, wherein the number of carbon atoms of the alkyl or alkenyl group in the component (A) is preferably 21 or more and 23 or less, more preferably 22.

<3> The oil-in-water emulsion composition according to <1> or <2>, wherein the average molar number of ethylene oxide added in the component (A) is preferably 1.5 or more and 3 or less, more preferably 1.5 or more and 2.5 or less.

<4> The oil-in-water emulsion composition according to any of <1> to <3>, wherein the component (A) is preferably at least one selected from the group consisting of polyoxyethylene (2) arachyl ether, polyoxyethylene (3) arachyl ether, polyoxyethylene (4) arachyl ether, polyoxyethylene (2) behenyl ether, polyoxyethylene (3) behenyl ether, polyoxyethylene (4) behenyl ether, polyoxyethylene (2) carnaubyl ether, polyoxyethylene (3) carnaubyl ether, and polyoxyethylene (4) carnaubyl ether, more preferably at least one selected from the group consisting of polyoxyethylene (2) behenyl ether, polyoxyethylene (3) behenyl ether, and polyoxyethylene (4) behenyl ether.

<5> The oil-in-water emulsion composition according to any of <1> to <4>, wherein the content of the component (A) with respect to the total amount of the composition is preferably 0.01% by mass or more, more preferably 0.1% by mass or more, even more preferably 0.5% by mass or more, and is preferably 15% by mass or less, more preferably 10% by mass or less, even more preferably 5% by mass or less.

<6> The oil-in-water emulsion composition according to any of <1> to <5>, wherein the content of the component (A) with respect to the total amount of the composition is preferably from 0.01 to 15% by mass, more preferably from 0.1 to 10% by mass, even more preferably from 0.5 to 5% by mass.

<7> The oil-in-water emulsion composition according to any of <1> to <6>, wherein the component (B) is other than a silicone oil and is preferably a liquid polar oil having an IOB value of 0.22 or more and 0.45 or less.

<8> The oil-in-water emulsion composition according to any of <1> to <7>, wherein the component (B) is preferably one or two or more selected from the group consisting of 2-ethylhexyl p-methoxycinnamate, propylene glycol isostearate, diethylhexyl sebacate, diisopropyl sebacate, and glyceryl tri-2-ethylhexanoate, more preferably one or two selected from 2-ethylhexyl p-methoxycinnamate and diisopropyl sebacate.

<9> The oil-in-water emulsion composition according to any of <1> to <8>, wherein the content of the component (B) with respect to the total amount of the composition is preferably 0.125% by mass or more, more preferably 0.25% by mass or more, even more preferably 1.25% by mass or more, and is preferably 30% by mass or less, more preferably 25% by mass or less, even more preferably 20% by mass or less.

<10> The oil-in-water emulsion composition according to any of <1> to <9>, wherein the content of the component (B) with respect to the total amount of the composition is preferably from 0.125 to 30% by mass, more preferably from 0.25 to 25% by mass, even more preferably from 1.25 to 20% by mass.

<11> The oil-in-water emulsion composition according to any of <1> to <10>, wherein the content of the component (B) with respect to the total amount of the liquid oil is preferably from 10 to 100% by mass, more preferably from 30 to 100% by mass, even more preferably from 50 to 100% by mass.

<12> The oil-in-water emulsion composition according to any of <1> to <11>, wherein the content mass ratio of the component (A) to the component (B), (A/B), is preferably 0.01 or more, more preferably 0.03 or more, even more preferably 0.04 or more, and is preferably 0.7 or less, more preferably 0.5 or less, even more preferably 0.4 or less.

<13> The oil-in-water emulsion composition according to any of <1> to <12>, wherein the content mass ratio of the component (A) to the component (B), (A/B), is preferably from 0.01 to 0.7, more preferably from 0.03 to 0.5, even more preferably from 0.04 to 0.4.

<14> The oil-in-water emulsion composition according to any of <1> to <13>, wherein the component (C) is preferably one or two or more selected from the group consisting of 2-ethylhexyl dimethoxybenzylidene dioxoimidazolidine propionate, diethylamino hydroxybenzoyl hexyl benzoate, 2,4-bis{[4-(2-ethylhexyloxy)-2-hydroxy]-phenyl}-6-(4-methoxyphenyl)-1,3,5-triazine, 2,4,6-tris[4-(2-ethylhexyloxycarbonyl)anilino]-1,3,5-triazine, and 4-tert-butyl-4'-methoxydibenzoylmethane, more preferably one or two or more selected from the group consisting of diethylamino hydroxybenzoyl hexyl benzoate, 2,4-bis{[4-(2-ethylhexyloxy)-2-hydroxy]-phenyl}-6-(4-methoxyphenyl)-1,3,5-triazine, and 2,4,6-tris[4-(2-ethylhexyloxycarbonyl)anilino]-1,3,5-triazine.

<15> The oil-in-water emulsion composition according to any of <1> to <14>, wherein the content of the component (C) with respect to the total amount of the composition is preferably 0.5% by mass or more, and is preferably 10% by mass or less, more preferably 7.5% by mass or less, even more preferably 5% by mass or less.

<16> The oil-in-water emulsion composition according to any of <1> to <15>, wherein the content of the component (C) with respect to the total amount of the composition is preferably from 0.5 to 10% by mass, more preferably from 0.5 to 7.5% by mass, even more preferably from 0.5 to 5% by mass.

<17> The oil-in-water emulsion composition according to any of <1> to <16>, wherein the content mass ratio of the component (B) to the component (C), (B/C), is preferably 2 or more, more preferably 2.2 or more, even more preferably 2.4 or more, and is preferably 20 or less, more preferably 10 or less, even more preferably 4 or less.

<18> The oil-in-water emulsion composition according to any of <1> to <17>, wherein the content mass ratio of the component (B) to the component (C), (B/C), is preferably from 2 to 20, more preferably from 2.2 to 10, even more preferably from 2.4 to 4.

<19> The oil-in-water emulsion composition according to any of <1> to <18>, further comprising (D) a water-soluble polymer.

<20> The oil-in-water emulsion composition according to <19>, wherein the water-soluble polymer (D) is preferably one or two or more selected from the group consisting of a carboxyvinyl polymer, an acrylic acid-alkyl methacrylate copolymer, xanthan gum, hydroxypropylmethylcellulose, polyacrylamide, a (sodium acrylate/sodium acryloyldimethyl taurate) copolymer, and hyaluronic acid or an alkali metal salt thereof.

<21> The oil-in-water emulsion composition according to <19> or <20>, wherein the content of the component (D) with respect to the total amount of the composition is preferably 0.01% by mass or more, more preferably 0.05% by mass or more, and is preferably 5% by mass or less, more preferably 3% by mass or less.

<22> The oil-in-water emulsion composition according to any of <1> to <21>, further comprising (E) a saturated monohydric alcohol having 1 to 3 carbon atoms.

<23> The oil-in-water emulsion composition according to <22>, wherein the content of the component (E) with respect to the total amount of the composition is preferably 1% by mass or more, more preferably 3% by mass or more, even more preferably 5% by mass or more, and is preferably 25% by mass or less, more preferably 20% by mass or less, even more preferably 15% by mass or less.

<24> The oil-in-water emulsion composition according to any of <1> to <23>, further comprising (F) a polyhydric alcohol.

<25> The oil-in-water emulsion composition according to <24>, wherein the content of the component (F) with respect to the total amount of the composition is preferably 0.1% by mass or more, more preferably 0.5% by mass or more, even more preferably 1% by mass or more, and is preferably 30% by mass or less, more preferably 20% by mass or less, even more preferably 10% by mass or less.

<26> The oil-in-water emulsion composition according to any of <1> to <25>, wherein the composition is preferably used for preventing sunburn.

<27> The oil-in-water emulsion composition according to any of <1> to <26>, wherein the composition is obtained preferably by dissolving an oil-phase component comprising the components (A) to (C) by heating at a temperature exceeding 45°C, preferably of 50 to 80°C and emulsifying the oil-phase component with an aqueous-phase component of 15 to 40°C, preferably 20 to 30°C.

<28> The oil-in-water emulsion composition according to <27>, wherein the emulsification is preferably performed by the gradual addition of the oil-phase component into the aqueous-phase component.

<29> Use of an oil-in-water emulsion composition according to any of <1> to <28> as a sunscreen skin preparation for external use.

<30> A method for producing an oil-in-water emulsion composition, comprising dissolving an oil-phase component comprising the following components (A) to (C) by heating at a temperature exceeding 45°C and emulsifying the oil-phase component with an aqueous-phase component having a temperature of 15 to 40°C:

(A) a polyoxyethylene alkyl or alkenyl ether which has an alkyl or alkenyl group having 20 or more and 24 or less carbon atoms and has an average molar number of ethylene oxide added of 1.5 or more and 4 or less,
(B) a polar oil which has an IOB value of 0.2 or more and 0.45 or less and is liquid at 25°C, provided that a silicone oil having a silicon backbone is excluded, and
(C) an ultraviolet absorber which is solid at 25°C,

wherein the content mass ratio of the component (A) to the component (B), (A/B), is 0.01 or more and 1.8 or less.

<31> The method for producing an oil-in-water emulsion composition according to <30>, wherein the emulsification is performed by the gradual addition of the oil-phase component into the aqueous-phase component.

Examples

Hereinafter, the present invention will be described in more detail with reference to Examples. However, the present invention is not intended to be limited by them.

Methods for water resistance test and sensory evaluation used in Examples and Comparative Examples below will be described for illustrative purposes.

(1) Structure formation test

Each sample shown in Table 1 was prepared. Emulsified particles were taken out of the obtained oil-in-water emulsion composition and visually observed to evaluate structure formation under the following criteria.

[Criteria for evaluating structure formation]

3: The emulsified particles do not collapse, and the formation of the structure can be confirmed in the neighborhood of the oil phase interface.
2: The emulsified particles readily collapse, though the formation of the structure can be confirmed in the neighborhood of the oil phase interface.
1: The formation of the structure can be confirmed in the whole oil phase, though the particles do not collapse.

- : No structure can be confirmed in the oil phase.

(2) Test on occluding properties

A hydrophilic membrane filter (MF-Millipore GSWP02500, manufactured by Millipore Japan Co., Ltd.; pore diameter: 0.22 $\mu$m, the filter was cut into a diameter of 15 mm and then used) was added to 100 $\mu$l of each sample shown in Table 2 or 3. The filter was thoroughly dried on a hot plate of 37°C to prepare a dried membrane filter of the sample. 5 g of pure water was placed in a cell, which was then covered with the dried membrane filter of the sample. Change in weight was measured at a humidity of 30% and a temperature of 30°C. The results were plotted as absolute values when n in (nX + m wherein X represents a time (h)) calculated by the least-squares method was defined as a water evaporation rate (unit: mg/h). A mean of three measurements per sample was determined. In this context, the water evaporation rate means that evaporation is more suppressed at a smaller water evaporation rate.

(3) Stability test

The presence or absence of deposition of crystals in each sample stored at 0°C for 1 month was confirmed visually and under a polarizing microscope (ECLIPSE 80i, manufactured by Nikon Corp.). Evaluation was conducted according to the following criteria for determining the deposition of crystals:

[Criteria for determining deposition of crystals]

3: Crystals were absent.
2: Crystals were slightly present.
1: Crystals were present.

(4) Test on UV protective ability

Each sample was separately applied at a proportion of 2 mg/cm$^2$ onto a PMMA sheet and then dried. Then, the ultraviolet protective ability was measured using an SPF analyzer (SPF 290S plus, manufactured by Optometricus USA, Inc.) in 8 predetermined areas of the sample on the PMMA sheet. The UV protective ability of each sample compared with the sample of Example 9 was determined according to the following expression, and evaluation was conducted according to evaluation criteria given below:

```
UV protective ability (%) = (Average SPF value of

the sample) / (Average SPF value of the sample of Example

9) × 100.
```

[Criteria for evaluating rate of improvement in UV protective ability]

3: 90% or more
2: 70% or more and less than 90%

1: Less than 70%

(5) Sensory evaluation

10 expert panelists actually used each sample shown in Table 2 or 3 and evaluated its usage characteristics [spreadability and staying property (uniform spreading without unevenness) and the presence or absence of a feeling of moisture, a feeling of stickiness, and a feeling of friction] according to evaluation criteria given below. Average scores are shown in the tables.

(Evaluation criteria)

(a) Spreadability and staying property

5: Very good spreadability and staying property
4: Good spreadability and staying property
3: Fair spreadability and staying property
2: Poor spreadability and staying property
1: Very poor spreadability and staying property

(b) Feeling of moisture

5: Very strong moist feeling of the skin
4: Strong moist feeling of the skin
3: Fair
2: Weak moist feeling of the skin
1: Very weak moist feeling of the skin

(c) Feeling of stickiness

5: Very weak feeling of stickiness
4: Weak feeling of stickiness
3: Fair
2: Strong feeling of stickiness
1: Very strong feeling of stickiness

(d) Feeling of friction

5: Very weak feeling of friction
4: Weak feeling of friction
3: Fair
2: Strong feeling of friction
1: Very strong feeling of friction

[Study on type of polyoxyethylene alkyl ether]

Example 1 and Comparative Examples 1 to 5

Each oil-in-water sunscreen was prepared according to the formulation shown in Table 1. The structure formation tests described above were carried out using these samples. The test results are also shown in Table 1.

[Table 1]

| Component | | | Example 1 | Comparative Example 1 | Comparative Example 2 | Comparative Example 3 | Comparative Example 4 | Comparative Example 5 |
|---|---|---|---|---|---|---|---|---|
| 1 | A | Polyoxyethylene (2) behenyl ether | 1 | | | | | |
| 2 | | Polyoxyethylene (5) behenyl ether | | 1 | | | | |
| 3 | | Polyoxyethylene (2) stearyl ether | | | 1 | | | |
| 4 | | Polyoxyethylene (2) cetyl ether | | | | 1 | | |
| 5 | | Polyoxyethylene (2) lauryl ether | | | | | 1 | |
| 6 | | Behenyl alcohol | | | | | | 1 |
| 7 | B | 2-Ethylhexyl p-methoxycinnamate *1 | 20 | 20 | 20 | 20 | 20 | 20 |
| 8 | C | Diethylamino hydroxybenzoyl hexyl benzoate *2 | 3 | 3 | 3 | 3 | 3 | 3 |
| 9 | | Pure water | Balance | Balance | Balance | Balance | Balance | Balance |
| | Evaluation | Structure formation | 3 | 2 | - | - | - | 1 |

*1: Uvinul MC-80 (manufactured by BASF Japan Ltd.)
*2: Uvinul A PLUS (manufactured by BASF Japan Ltd.)

EP 2 921 163 B1

(Production method)

a: The components (1) to (8) are dissolved by heating at 80°C and uniformly mixed.
b: The mixture obtained in the step a was added dropwise to the component (9) by way of a dropper.

In the oil-in-water emulsion composition of Example 1, the structure was formed on the oil phase interface. The central part of the capsule was composed of 2-ethylhexyl p-methoxycinnamate and diethylamino hydroxybenzoyl hexyl benzoate, demonstrating that the polyoxyethylene alkyl ether (A) is involved in the formation of the structure. On the other hand, in the samples of Comparative Examples 1 to 4 in which the polyoxyethylene alkyl ether (A) was replaced with a polyoxyethylene alkyl ether other than the polyoxyethylene alkyl ether (A), no structure was confirmed in the oil phase. Also, in the sample of Comparative Example 5 in which the polyoxyethylene alkyl ether (A) was replaced with behenyl alcohol, solidification occurred not only on the oil phase interface but in the whole oil phase, and no structure was able to be confirmed in the neighborhood of the interface.

[Study on polar oil]

Examples 2 to 8 and Comparative Examples 6 to 9

Each oil-in-water sunscreen was prepared according to the formulation shown in Table 2. The test on occluding properties, the stability test, and the sensory evaluation were carried out using these samples. The test results are also shown in Table 2.

## [Table 2]

| | | Component | Example 2 | Example 3 | Example 4 | Example 5 | Example 6 | Example 7 | Example 8 | Comparative Example 6 | Comparative Example 7 | Comparative Example 8 | Comparative Example 9 |
|---|---|---|---|---|---|---|---|---|---|---|---|---|---|
| 1 | A | Polyoxyethylene (2) behenyl ether | 1 | 1 | 1 | 1 | 1 | 1 | 1 | 1 | 1 | | 1 |
| 2 | B | 2-Ethylhexyl p-methoxycinnamate *1 | 12 | | | | | 12 | 12 | | | 12 | |
| 3 | B | Propylene glycol isostearate | | 12 | | | | | | | | | |
| 4 | B | Diethylhexyl sebacate | | | 12 | | | | | | | | |
| 5 | B | Diisopropyl sebacate | | | | 12 | | | | | | | |
| 6 | B | Glyceryl tri-2-ethylhexanoate | | | | | 12 | | | | | | |
| 7 | | Liquid paraffin | | | | | | | | 12 | | | |
| 8 | | Polyethylene glycol diisostearate | | | | | | | | | 12 | | |
| 9 | C | Diethylamino hydroxybenzoyl hexyl benzoate *2 | 3 | 3 | 3 | 3 | 3 | | | 3 | 3 | 3 | 3 |
| 10 | C | 2,4-Bis-{[4-(2-ethylhexyloxy)-2-hydroxy]-phenyl}-6-(4-methoxyphenyl)-1,3,5-triazine *3 | | | | | | 3 | | | | | |
| 11 | C | 2,4,6-Tris[4-(2-ethylhexyloxycarbonyl)anilino]-1,3,5-triazine *4 | | | | | | | 3 | | | | |
| 12 | F | Glycerin | 0.5 | 0.5 | 0.5 | 0.5 | 0.5 | 0.5 | 0.5 | 0.5 | 0.5 | 0.5 | 0.5 |
| 13 | F | 1,3-Butylene glycol | 1 | 1 | 1 | 1 | 1 | 1 | 1 | 1 | 1 | 1 | 1 |
| 14 | E | Ethanol | 12 | 12 | 12 | 12 | 12 | 12 | 12 | 12 | 12 | 12 | 12 |
| 15 | D | (Sodium acrylate/sodium acryloyldimethyl taurate) copolymer *5 | 2.5 | 2.5 | 2.5 | 2.5 | 2.5 | 2.5 | 2.5 | 2.5 | 2.5 | 2.5 | 2.5 |
| 16 | D | Xanthan gum | 0.1 | 0.1 | 0.1 | 0.1 | 0.1 | 0.1 | 0.1 | 0.1 | 0.1 | 0.1 | 0.1 |
| 17 | | Disodium edetate | 0.02 | 0.02 | 0.02 | 0.02 | 0.02 | 0.02 | 0.02 | 0.02 | 0.02 | 0.02 | 0.02 |
| 18 | | Pure water | Balance | Balance | Balance | Balance | Balance | Balance | Balance | Balance | Balance | Balance | Balance |
| Evaluation | | Occluding effect (water evaporation rate) | 9.4 | 9.5 | 9.5 | 9.6 | 9.3 | 9.4 | 9.5 | 9.8 | 9.7 | 10 | - |
| Evaluation | | Stability (visual confirmation of presence or absence of deposition) | 3 | 3 | 3 | 3 | 3 | 3 | 3 | 1 | 1 | 3 | - |
| Evaluation | | Stability (confirmation of presence or absence of deposition under polarizing microscope) | 3 | 2 | 2 | 3 | 2 | 3 | 3 | 1 | 1 | 2 | - |
| Evaluation | | Spreadability and staying property | 4.5 | 4.6 | 4.5 | 4.8 | 4.6 | 4.6 | 4.4 | 3.8 | 3.6 | 3.4 | - |
| Evaluation | | Feeling of moisture | 4.2 | 4.3 | 4.2 | 4.3 | 4.2 | 4.2 | 4.2 | 4.3 | 4.3 | 4.2 | - |
| Evaluation | | Feeling of stickiness | 4.4 | 4.5 | 4.6 | 4.6 | 4.4 | 4.5 | 4.6 | 3.8 | 4 | 3.5 | - |
| Evaluation | | Feeling of friction | 4.2 | 4.3 | 4.2 | 4.2 | 4.3 | 4.2 | 4.3 | 4.3 | 4.2 | 4.2 | - |

*3 Tinosorb S (manufactured by BASF Japan Ltd.)

*4 Uvinul T-150 (manufactured by BASF Japan Ltd.)

*5 SIMULGEL EG (manufactured by SEPPIC)

(Production method)

a: The components (1) to (11) are dissolved by heating at 70°C and uniformly mixed.
b: The components (12) to (18) are uniformly mixed at room temperature (25°C ± 3°C).
c: While the product obtained in the step b is stirred, the product obtained in the step a is gradually added thereto. The mixture is uniformly mixed.
d: The mixture obtained in the step c is cooled to room temperature.

As is evident from Table 2, the oil-in-water emulsion compositions comprising the components of the present invention were visually confirmed to be free from deposition and were also excellent in feeling upon use. On the other hand, the samples of Comparative Examples 6 and 7 in which the component (B) was replaced with an oil agent other than the component (B) were confirmed to have the deposition and received lower evaluations than those of the samples of Examples as to the occluding effect and the feeling upon use. Also, in the sample of Comparative Example 8 in which the polyoxyethylene alkyl ether (A) was replaced with 2-ethylhexyl p-methoxycinnamate, no structure was confirmed. In addition, this sample had the deposition of the component (C) and received lower evaluations than those of the samples of Examples as to the occluding effect and the feeling upon use. Alternatively, the sample of Comparative Example 9 was separated and was thus not evaluable.

[Study on content ratio]

Examples 9 to 20

Each oil-in-water sunscreen was prepared according to the formulation shown in Table 3. The test on occluding properties, the stability test, the test on UV protective ability, and the sensory evaluation test were carried out using these samples. The test results are also shown in Table 3. Even the samples having lower UV protective ability compared with the sample of Example 9 in the test on UV protective ability in Table 3 can be preferably used as sunscreens for daily use which does not require much UV protective ability.

[Table 3]

| | | Component | Example 9 | Example 10 | Example 11 | Example 12 | Example 13 | Example 14 | Example 15 | Example 16 | Example 17 | Example 18 | Example 19 | Example 20 |
|---|---|---|---|---|---|---|---|---|---|---|---|---|---|---|
| 1 | A | Polyoxyethylene (2) behenyl ether | 1 | 2 | 0.2 | 3 | 4 | 1 | 0.2 | 6 | 1 | 1 | 1 | 1 |
| 2 | B | 2-Ethylhexyl p-methoxycinnamate *1 | 7.5 | 7.5 | 7.5 | 6 | 6 | 15 | 15 | 3 | 6 | 7.5 | 9 | 7.5 |
| 3 | C | Diethylamino hydroxybenzoyl hexyl benzoate *2 | 2.5 | 2.5 | 2.5 | 2.5 | 2.5 | 2.5 | 3 | 1 | 3 | 0.5 | 5 | 0.3 |
| 4 | F | Glycerin | 0.5 | 0.5 | 0.5 | 0.5 | 0.5 | 0.5 | 0.5 | 0.5 | 0.5 | 0.5 | 0.5 | 0.5 |
| 5 | F | 1,3-Butylene glycol | 1 | 1 | 1 | 1 | 1 | 1 | 1 | 1 | 1 | 1 | 1 | 1 |
| 6 | E | Ethanol | 12 | 12 | 12 | 12 | 12 | 12 | 12 | 12 | 12 | 12 | 12 | 12 |
| 7 | D | (Sodium acrylate/sodium acryloyldimethyl taurate) copolymer *5 | 2.5 | 2.5 | 2.5 | 2.5 | 2.5 | 2.5 | 2.5 | 2.5 | 2.5 | 2.5 | 2.5 | 2.5 |
| 8 | D | Xanthan gum | 0.1 | 0.1 | 0.1 | 0.1 | 0.1 | 0.1 | 0.1 | 0.1 | 0.1 | 0.1 | 0.1 | 0.1 |
| 9 | | Dimethylpolysiloxane (6 mm$^2$/s) *6 | 5.5 | 5.5 | 5.5 | 5.5 | 5.5 | 5.5 | 5.5 | 5.5 | 5.5 | 5.5 | 5.5 | 5.5 |
| 10 | | Hyaluronic acid | 0.02 | 0.02 | 0.02 | 0.02 | 0.02 | 0.02 | 0.02 | 0.02 | 0.02 | 0.02 | 0.02 | 0.02 |
| 11 | | Alga extract *7 | 0.1 | 0.1 | 0.1 | 0.1 | 0.1 | 0.1 | 0.1 | 0.1 | 0.1 | 0.1 | 0.1 | 0.1 |
| 12 | | Hydrolyzed collagen *8 | 0.1 | 0.1 | 0.1 | 0.1 | 0.1 | 0.1 | 0.1 | 0.1 | 0.1 | 0.1 | 0.1 | 0.1 |
| 13 | | Royal jelly extract *9 | 0.1 | 0.1 | 0.1 | 0.1 | 0.1 | 0.1 | 0.1 | 0.1 | 0.1 | 0.1 | 0.1 | 0.1 |
| 14 | | Job's tears (*Coix lacryma-jobi* var. *ma-yuen*) seed extract *10 | 0.1 | 0.1 | 0.1 | 0.1 | 0.1 | 0.1 | 0.1 | 0.1 | 0.1 | 0.1 | 0.1 | 0.1 |
| 15 | | Firethorn (*Pyracantha fortuneana*) extract *11 | 0.1 | 0.1 | 0.1 | 0.1 | 0.1 | 0.1 | 0.1 | 0.1 | 0.1 | 0.1 | 0.1 | 0.1 |
| 16 | | Watercress (*Nasturtium officinale*) extract *12 | 0.1 | 0.1 | 0.1 | 0.1 | 0.1 | 0.1 | 0.1 | 0.1 | 0.1 | 0.1 | 0.1 | 0.1 |
| 17 | | Disodium edetate | 0.02 | 0.02 | 0.02 | 0.02 | 0.02 | 0.02 | 0.02 | 0.02 | 0.02 | 0.02 | 0.02 | 0.02 |
| 18 | | Pure water | Balance | Balance | Balance | Balance | Balance | Balance | Balance | Balance | Balance | Balance | Balance | Balance |
| | | (A)/(B) | 0.133 | 0.267 | 0.027 | 0.500 | 0.667 | 0.067 | 0.013 | 2.000 | 0.167 | 0.133 | 0.111 | 0.133 |
| | | (B)/(C) | 3.0 | 3.0 | 3.0 | 2.4 | 2.4 | 6.0 | 5.0 | 3.0 | 2.0 | 15.0 | 1.8 | 25.0 |
| | Evaluation | Occluding effect (water evaporation rate) | 9.2 | 9.1 | 9.5 | 9.0 | 8.9 | 9.3 | 9.3 | 6.5 | 8.9 | 9.0 | 9.2 | 9.1 |
| | | Stability (visual confirmation of presence or absence of deposition) | 3 | 3 | 3 | 3 | 3 | 3 | 3 | 3 | 3 | 3 | 3 | 3 |
| | | Stability (confirmation of presence or absence of deposition under polarizing microscope) | 3 | 3 | 2 | 3 | 3 | 3 | 3 | 3 | 3 | 3 | 3 | 3 |
| | | UV protective ability | 3 | 3 | 3 | 3 | 3 | 3 | 3 | 1 | 2 | 2 | 3 | 2 |
| | | Spreadability and staying property | 4.5 | 4.3 | 4.5 | 4.2 | 4.3 | 4.1 | 4 | 3.8 | 4.5 | 4.3 | 4.2 | 4.2 |
| | | Feeling of moisture | 4.2 | 4.4 | 4.2 | 4.5 | 4.2 | 4.6 | 4.4 | 4.7 | 4.3 | 4.4 | 4 | 4.3 |
| | | Feeling of stickiness | 4.4 | 4.2 | 4.2 | 4.3 | 4.6 | 4 | 3.6 | 4 | 4.2 | 4.4 | 3.2 | 4.8 |
| | | Feeling of friction | 4.2 | 4.4 | 4.5 | 4.2 | 4.2 | 4.8 | 4.6 | 4 | 4 | 4.2 | 4 | 4.3 |

*6:  Silicone KF-96A (6CS) (manufactured by Shin-Etsu Chemical Co., Ltd.)
*7:  Alga Extract M (manufactured by Maruzen Pharmaceuticals Co., Ltd.)
*8:  PROMOIS WU-32R (manufactured by Seiwa Kasei Co., Ltd.)
*9:  Royal Jelly Extract (manufactured by Ichimaru Pharcos Co., Ltd.)
*10  Coix Seed Extract BG-S (manufactured by Maruzen Pharmaceuticals Co., Ltd.)
*11  Firethorn (manufactured by Maruzen Pharmaceuticals Co., Ltd.)
*12  WaterCress-KB (manufactured by Silab)

EP 2 921 163 B1

(Production method)

a: The components (1) to (3) and (9) are dissolved by heating at 70°C and uniformly mixed.
b: The components (4) to (8) and (10) to (18) are uniformly mixed at room temperature (25°C $\pm$ 3°C).
c: While the product obtained in the step b is stirred, the product obtained in the step a is gradually added thereto.
d: The mixture obtained in the step c is cooled to room temperature.

Hereinafter, Formulation Example of the emulsion composition of the present invention will be given. The formulation is expected to spread and stay well, offer reduced feelings of friction and stickiness, be excellent in stability and UV protective ability, and also exhibit an excellent moisturizing effect.

Formulation Example 1 (oil-in-water sunscreen) Components

| | Content (% by mass) |
|---|---|
| [Component (A)] | |
| Polyoxyethylene (2) behenyl ether | 1 |
| [Component (C)] | |
| Diethylamino hydroxybenzoyl hexyl benzoate | 2 |
| 2,4-Bis{[4-(2-ethylhexyloxy)-2-hydroxy]-phenyl}-6-(4-methoxyphenyl)-1,3,5-triazine | 1 |
| 2,4,6-Tris[4-(2-ethylhexyloxycarbonyl)anilino]-1,3,5-triazine | 0.5 |
| [Component (B)] | |
| 2-Ethylhexyl p-methoxycinnamate | 7.5 |
| [Component F] | |
| Glycerin | 0.5 |
| 1,3-Butylene glycol | 1 |
| [Component E] | |
| Ethanol | 10 |
| [Component (D)] | |
| (Sodium acrylate/sodium acryloyldimethyl taurate) copolymer | 2.5 |
| Xanthan gum | 0.1 |
| [Other components] | |
| Dimethylpolysiloxane (6 mm$^2$/s) | 3.0 |
| Disodium edetate | 0.02 |
| Phenoxyethanol | 0.1 |
| Pure water | Balance |

**Claims**

1.   An oil-in-water emulsion composition comprising the following components (A) to (C):

(A) a polyoxyethylene alkyl or alkenyl ether which has an alkyl or alkenyl group having 20 or more and 24 or less carbon atoms and has an average molar number of ethylene oxide added of 1.5 or more and 4 or less,
(B) a polar oil which has an IOB value of 0.2 or more and 0.45 or less and is liquid at 25°C, provided that a silicone oil having a silicon backbone is excluded, and
(C) an ultraviolet absorber which is solid at 25°C, wherein the content mass ratio of the component (A) to the component (B), (A/B), is 0.01 or more and 1.8 or less.

2.   The oil-in-water emulsion composition according to claim 1, wherein the content mass ratio of the component (B) to the component (C), (B/C), is 2 or more and 20 or less.

3.   The oil-in-water emulsion composition according to claim 1 or 2, further comprising a liquid oil other than the component (B), wherein the content of the component (B) with respect to the total amount of the liquid oil is 50% by mass or more.

4. The oil-in-water emulsion composition according to any one of claims 1 to 3, wherein the component (B) is a liquid polar oil having an IOB value of 0.22 or more and 0.45 or less.

5. The oil-in-water emulsion composition according to any one of claims 1 to 4, wherein the component (B) is one or two or more selected from the group consisting of 2-ethylhexyl p-methoxycinnamate, propylene glycol isostearate, diethylhexyl sebacate, diisopropyl sebacate, and glyceryl tri-2-ethylhexanoate.

6. The oil-in-water emulsion composition according to any one of claims 1 to 5, wherein the component (C) is one or two or more selected from the group consisting of diethylamino hydroxybenzoyl hexyl benzoate, 2,4-bis{[4-(2-ethylhexyloxy)-2-hydroxy]phenyl}-6-(4-methoxyphenyl)-1,3,5-triazine, and 2,4,6-tris[4-(2-ethylhexyloxycarbonyl)anilino]-1,3,5-triazine.

7. The oil-in-water emulsion composition according to any one of claims 1 to 6, wherein the component (A) is at least one selected from the group consisting of polyoxyethylene (2) arachyl ether, polyoxyethylene (3) arachyl ether, polyoxyethylene (4) arachyl ether, polyoxyethylene (2) behenyl ether, polyoxyethylene (3) behenyl ether, polyoxyethylene (4) behenyl ether, polyoxyethylene (2) carnaubyl ether, polyoxyethylene (3) carnaubyl ether, and polyoxyethylene (4) carnaubyl ether.

8. The oil-in-water emulsion composition according to any one of claims 1 to 7, further comprising (D) a water-soluble polymer.

9. The oil-in-water emulsion composition according to claim 8, wherein the water-soluble polymer (D) is one or two or more selected from the group consisting of a carboxyvinyl polymer, an acrylic acid-alkyl methacrylate copolymer, xanthan gum, hydroxypropylmethylcellulose, polyacrylamide, a (sodium acrylate/sodium acryloyldimethyl taurate) copolymer, and hyaluronic acid or an alkali metal salt thereof.

10. The oil-in-water emulsion composition according to any one of claims 1 to 9, wherein the content mass ratio of the component (A) to the component (B), (A/B), is 0.01 or more and 0.7 or less.

11. The oil-in-water emulsion composition according to any one of claims 1 to 10, wherein the content mass ratio of the component (B) to the component (C), (B/C), is 2.2 or more and 10 or less.

12. Oil-in-water emulsion composition according to any one of claims 1 to 11 for external use as a sunscreen skin preparation.

13. A method for producing an oil-in-water emulsion composition, comprising dissolving an oil-phase component comprising the following components (A) to (C) by heating at a temperature exceeding 45°C and emulsifying the oil-phase component with an aqueous-phase component having a temperature of 15 to 40°C:

(A) a polyoxyethylene alkyl or alkenyl ether which has an alkyl or alkenyl group having 20 or more and 24 or less carbon atoms and has an average molar number of ethylene oxide added of 1.5 or more and 4 or less,
(B) a polar oil which has an IOB value of 0.2 or more and 0.45 or less and is liquid at 25°C, provided that a silicone oil having a silicon backbone is excluded, and
(C) an ultraviolet absorber which is solid at 25°C,

wherein the content mass ratio of the component (A) to the component (B), (A/B), is 0.01 or more and 1.8 or less.

14. The method for producing an oil-in-water emulsion composition according to claim 13, wherein the emulsification is performed by the gradual addition of the oil-phase component into the aqueous-phase component.

**Patentansprüche**

1. Öl-in-Wasser-Emulsionszusammensetzung, enthaltend die folgenden Komponenten (A) bis (C):

(A) einen Polyoxyethylenalkyl- oder -alkenylether, der eine Alkyl- oder Alkenylgruppe mit 20 oder mehr und 24 oder weniger Kohlenstoffatomen aufweist und eine durchschnittliche molare Zahl von zugegebenem Ethylenoxid von 1,5 oder mehr und 4 oder weniger hat,

(B) ein polares Öl, das einen IOB-Wert von 0,2 oder mehr und 0,45 oder weniger hat und bei 25°C flüssig ist, vorausgesetzt, dass ein Silikonöl mit einem Silikonrückgrat ausgeschlossen ist, und

(C) einen Ultraviolettabsorber, der bei 25°C fest ist, worin das Masseverhältnis des Gehaltes der Komponente (A) zu der Komponenten (B), (A/B), 0,01 oder mehr und 1,8 oder weniger ist.

2. Öl-in-Wasser-Emulsionszusammensetzung gemäß Anspruch 1, worin das Masseverhältnis des Gehaltes der Komponente (B) zu der Komponenten (C), (B/C), 2 oder mehr und 20 oder weniger ist.

3. Öl-in-Wasser-Emulsionszusammensetzung gemäß Anspruch 1 oder 2, weiterhin enthaltend ein anderes flüssiges Öl als die Komponente (B), worin der Gehalt der Komponente (B) in Bezug auf die Gesamtmenge des flüssigen Öls 50 Masse-% oder mehr ist.

4. Öl-in-Wasser-Emulsionszusammensetzung gemäß einem der Ansprüche 1 bis 3, worin die Komponente (B) ein flüssiges polares Öl mit einem IOB-Wert von 0,22 oder mehr und 0,45 oder weniger ist.

5. Öl-in-Wasser-Emulsionszusammensetzung gemäß einem der Ansprüche 1 bis 4, worin die Komponente (B) ein oder zwei oder mehrere ist, ausgewählt aus der Gruppe, bestehend aus 2-Ethylhexyl-p-methoxycinnamat, Propylenglykolisostearat, Diethylhexylsebacat, Diisopropylsebacat und Glyceryl-tri-2-ethylhexanoat.

6. Öl-in-Wasser-Emulsionszusammensetzung gemäß einem der Ansprüche 1 bis 5, worin die Komponente (C) eine oder zwei oder mehrere ist, ausgewählt aus der Gruppe, bestehend aus Diethylaminohydroxybenzoylhexylbenzoat, 2,4-Bis{[4-(2-ethylhexyloxy)-2-hydroxylphenyl}-6-(4-methoxyphenyl)-1,3,5-triazin und 2,4,6-Tris[4-(2-ethylhexyloxycarbonyl)anilin]-1,3,5-triazin.

7. Öl-in-Wasser-Emulsionszusammensetzung gemäß einem der Ansprüche 1 bis 6, worin die Komponente (A) zumindest eines ist, ausgewählt aus der Gruppe, bestehend aus Polyoxyethylen-(2)-arachylether, Polyoxyethylen-(3)-arachylether, Polyoxyethylen-(4)-arachylether, Polyoxyethylen-(2)-behenylether, Polyoxyethylen-(3)-behenylether, Polyoxyethylen-(4)-behenylether, Polyoxyethylen-(2)-carnaubylether, Polyoxyethylen-(3)-carnaubylether und Polyoxyethylen-(4)-carnaubylether.

8. Öl-in-Wasser-Emulsionszusammensetzung gemäß einem der Ansprüche 1 bis 7, weiterhin enthaltend (D) ein wasserlösliches Polymer.

9. Öl-in-Wasser-Emulsionszusammensetzung gemäß Anspruch 8, worin das wasserlösliche Polymer (D) eins oder zwei oder mehrere ist, ausgewählt aus der Gruppe, bestehend aus einem Carboxyvinylpolymer, einem Acrylsäure-Alkylmethacrylat-Copolymer, Xanthangummi, Hydroxypropylmethylcellulose, Polyacrylamid, einem (Natriumacrylat/Natriumacryloyldimethyltaurat)-Copolymer und Hyaluronsäure oder einem Alkalimetallsalz davon.

10. Öl-in-Wasser-Emulsionszusammensetzung gemäß einem der Ansprüche 1 bis 9, worin das Masseverhältnis des Gehaltes der Komponente (A) zu der Komponente (B), (A/B), 0,01 oder mehr und 0,7 oder weniger ist.

11. Öl-in-Wasser-Emulsionszusammensetzung gemäß einem der Ansprüche 1 bis 10, worin das Masseverhältnis des Gehaltes der Komponente (B) zu der Komponente (C), (B/C), 2,2 oder mehr und 10 oder weniger ist.

12. Öl-in-Wasser-Emulsionszusammensetzung gemäß einem der Ansprüche 1 bis 11 zur externen Verwendung als Sonnenschutz-Hautpräparat.

13. Verfahren zur Erzeugung einer Öl-in-Wasser-Emulsionszusammensetzung, enthaltend das Auflösen einer Ölphasenkomponente, enthaltend die folgenden Komponenten (A) bis (C), durch Erwärmen bei einer Temperatur von mehr als 45°C und Emulgieren der Ölphasenkomponente mit einer Komponente der wässrigen Phase mit einer Temperatur von 15 bis 40°C:

(A) ein Polyoxyethylenalkyl- oder -alkenylether, der eine Alkyl- oder Alkenylgruppe mit 20 oder mehr und 24 oder weniger Kohlenstoffatomen aufweist und eine durchschnittliche molare Zahl von zugegebenem Ethylenoxid von 1,5 oder mehr und 4 oder weniger hat,

(B) ein polares Öl, das einen IOB-Wert von 0,2 oder mehr und 0,45 oder weniger hat und bei 25°C flüssig ist, vorausgesetzt, dass ein Silikonöl mit einem Silikonrückgrat ausgeschlossen ist, und

(C) ein Ultraviolettabsorber der bei 25°C fest ist, worin das Masseverhältnis des Gehaltes der Komponente (A) zu der Komponenten (B), (A/B), 0,01 oder mehr und 1,8 oder weniger ist.

14. Verfahren zur Erzeugung einer Öl-in-Wasser-Emulsionszusammensetzung gemäß Anspruch 13, worin die Emulgierung durch graduelle Addition der Ölphasenkomponente in die Komponente der wässrigen Phase durchgeführt wird.

**Revendications**

1. Composition d'émulsion huile-dans-eau comprenant les composants (A) à (C) suivants :

(A) un éther d'alkyle ou d'alcényle de polyoxyéthylène qui a un groupe alkyle ou alcényle ayant 20 ou plus et 24 ou moins atomes de carbone et qui a un nombre molaire moyen d'oxyde d'éthylène ajouté de 1,5 ou plus et de 4 ou moins,

(B) une huile polaire qui a un indice IOB de 0,2 ou plus et 0,45 ou moins et qui est liquide à 25°C, sous réserve de l'exclusion d'une huile de silicone ayant un squelette de silicium, et

(C) un absorbeur d'ultraviolets qui est solide à 25°C,

dans laquelle le rapport massique de teneurs du composant (A) sur le composant (B), (A/B), est de 0,01 ou plus et de 1,8 ou moins.

2. Composition d'émulsion huile-dans-eau selon la revendication 1, dans laquelle le rapport massique de teneurs du composant (B) sur le composant (C), (B/C), est de 2 ou plus et de 20 ou moins.

3. Composition d'émulsion huile-dans-eau selon la revendication 1 ou 2, comprenant en outre une huile liquide autre que le composant (B), dans laquelle la teneur du composant (B) par rapport à la quantité totale de l'huile liquide est de 50 % en masse ou plus.

4. Composition d'émulsion huile-dans-eau selon l'une quelconque des revendications 1 à 3, dans laquelle le composant (B) est une huile polaire liquide ayant un indice IOB de 0,22 ou plus et de 0,45 ou moins.

5. Composition d'émulsion huile-dans-eau selon l'une quelconque des revendications 1 à 4, dans laquelle le composant (B) est un ou deux éléments ou plus choisis dans le groupe constitué par le p-méthoxycinnamate de 2-éthylhexyle, l'isostéarate de propylèneglycol, le sébacate de diéthylhexyle, le sébacate de diisopropyle et le tri-2-éthylhexanoate de glycéryle.

6. Composition d'émulsion huile-dans-eau selon l'une quelconque des revendications 1 à 5, dans laquelle le composant (C) est un ou deux composés ou plus choisis dans le groupe constitué par le benzoate de diéthylaminohydroxybenzoylhexyle, le 2,4-bis{[4-(2-éthylhexyloxy)-2-hydroxy]phényl}-6-(4-méthoxyphényl)-1,3,5-triazine et la 2,4,6-tris[4-(2-éthylhexyloxycarbonyl)anilino]-1,3,5-triazine.

7. Composition d'émulsion huile-dans-eau selon l'une quelconque des revendications 1 à 6, dans laquelle le composant (A) est au moins un choisi parmi l'éther d'arachyle de polyoxyéthylène (2), l'éther d'arachyle de polyoxyéthylène (3), l'éther d'arachyle de polyoxyéthylène (4), l'éther de béhényle de polyoxyéthylène (2), l'éther de béhényle de polyoxyéthylène (3), l'éther de béhényle de polyoxyéthylène (4), l'éther de carnaubyle polyoxyéthylène (2), l'éther de carbaubyle de polyoxyéthylène (3) et l'éther de carnaubyle de polyoxyéthylène (4).

8. Composition d'émulsion huile-dans-eau selon l'une quelconque des revendications 1 à 7, comprenant en outre (D) un polymère hydrosoluble.

9. Composition d'émulsion huile-dans-eau selon la revendication 8, dans laquelle le polymère soluble dans l'eau (D) est un ou deux ou plusieurs éléments choisis dans le groupe constitué par un polymère carboxyvinylique, un copolymère acide acrylique-méthacrylate d'alkyle, la gomme de xanthane, l'hydroxypropylméthylcellulose, un polyacrylamide, un copolymère (acrylate de sodium/taurate d'acryloyldiméthyl) et l'acide hyaluronique, ou un sel de métal alcalin de ceux-ci.

**10.** Composition d'émulsion huile-dans-eau selon l'une quelconque des revendications 1 à 9, dans laquelle le rapport massique de teneurs du composant (A) sur le composant (B), (A/B), est de 0,01 ou plus et de 0,7 ou moins.

**11.** Composition d'émulsion huile-dans-eau selon l'une quelconque des revendications 1 à 10, dans laquelle le rapport massique de teneurs du composant (B) sur le composant (C), (B/C), est de 2,2 ou plus et de 10 ou moins.

**12.** Composition d'émulsion huile-dans-eau selon l'une quelconque des revendications 1 à 11 pour une utilisation externe comme préparation pour la peau de type écran solaire.

**13.** Procédé de fabrication d'une composition d'émulsion huile-dans-eau, comprenant la dissolution d'un composant de phase huileuse comprenant les composants (A) à (C) suivants en chauffant à une température supérieure à 45°C et en émulsifiant le composant de phase huileuse avec un composant de phase aqueuse ayant une température de 15 à 40°C :

(A) un éther d'alkyle ou d'alcényle de polyoxyéthylène qui a un groupe alkyle ou alcényle ayant 20 ou plus et 24 ou moins atomes de carbone et qui a un nombre molaire moyen d'oxyde d'éthylène ajouté de 1,5 ou plus et de 4 ou moins,
(B) une huile polaire ayant un indice IOB de 0,2 ou plus et de 0,45 ou moins et qui est liquide à 25°C, sous réserve de l'exclusion d'une huile de silicone ayant un squelette de silicium, et
(C) un absorbeur d'ultraviolets qui est solide à 25°C,

dans lequel le rapport massique de teneurs du composant (A) sur le composant (B), (A/B), est de 0,01 ou plus et de 1,8 ou moins.

**14.** Procédé de production d'une composition d'émulsion huile-dans-eau selon la revendication 13, dans lequel l'émulsification est effectuée par l'addition progressive du composant de phase huileuse dans le composant de phase aqueuse.

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3491933 B
- JP 2008162988 A
- WO 2007122822 A
- WO 2011136121 A
- WO 2011155404 A
- WO 2013161429 A
- EP 2543356 A

- JP 2007031336 A
- JP 2012001527 A
- JP H01266844 B
- JP 2011195478 A
- JP H10194923 B
- JP H07291827 B

**Non-patent literature cited in the description**

- Yuki Gainenzu - Kiso to Oyo. **YOSHIO KODA.** Organic Conceptual Diagram - Basis and Application - in English. Sankyo Publishing Co., Ltd, 1984, 11-17